(19) Europäisches Patentamt
European Patent Office
Office européen des brevets

(11) EP 0 778 287 B1

(12) FASCICULE DE BREVET EUROPEEN

(45) Date de publication et mention de la délivrance du brevet: **13.05.1998 Bulletin 1998/20**

(51) Int. Cl.[6]: **C08B 37/16**, A61K 7/00

(21) Numéro de dépôt: **96402446.7**

(22) Date de dépôt: **14.11.1996**

# (54) Dérivés de cyclodextrine

Cyclodextrin Derivate

Cyclodextrin derivatives

(84) Etats contractants désignés:
**CH DE ES FR GB IT LI NL**

(30) Priorité: **04.12.1995 FR 9514318**

(43) Date de publication de la demande:
**11.06.1997 Bulletin 1997/24**

(73) Titulaire: **L'OREAL**
**75008 Paris (FR)**

(72) Inventeurs:
- **Richard, Hervé**
**93420 Villepinte (FR)**
- **Leduc, Madeleine**
**75011 Paris (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
- **Aucun document pertinent relevé**

EP 0 778 287 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouveaux dérivés de cyclodextrine, leur procédé de préparation et leur utilisation en cosmétique.

Les cyclodextrines sont des oligosaccharides de formule

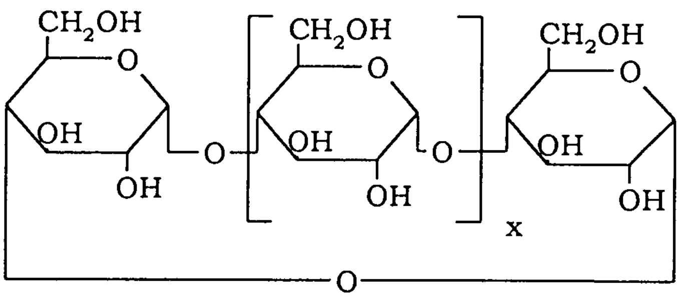

dans laquelle x peut être un nombre égal à 4 (ce qui correspond à l'α-cyclodextrine), 5 (β-cyclodextrine) ou 6 (γ-cyclodextrine).

Les cyclodextrines sont des composés bien connus, notamment pour leur aptitude à former des complexes d'inclusion avec des substances actives.

Par ailleurs, la possibilité de substituer un ou plusieurs groupements hydroxyles des cyclodextrines en vue de greffer des substances actives a été étudiée. Ainsi, l'article de BERGER, Journal of Organic Chemistry, 56, pages 3514-3520, 1991, décrit des β-cyclodextrines greffées d'un seul greffon de m,m'-disulfonyl benzophénone. L'article TABUSHI de Tetrahedron Letters n° 29, pages 2503-2506, 1977, décrit des β-cyclodextrines greffées d'un greffon benzophénone par une liaison ester.

Toutefois, ces composés greffés portent peu de greffons actifs ce qui limite leur activité et leur efficacité.

Après diverses recherches sur les cyclodextrines, la demanderesse a constaté de façon surprenante qu'il était possible d'obtenir des cyclodextrines ayant une activité filtrante et/ou antioxydante, avec un taux de greffage plus important permettant d'améliorer l'activité et l'efficacité de ces composés.

La présente invention a donc pour objet de nouveaux dérivés de cyclodextrine répondant à la formule générale (I) suivante :

$$CD\,(OH)_l\,(OR_1)_m(OR_2)_n \qquad (I)$$

dans laquelle

* CD représente le squelette de base de l'α-, β- ou γ-cyclodextrine sans les groupements hydroxyle,

* $OR_1$ représente un groupement, lié au squelette de base de la cyclodextrine, dans lequel $R_1$ représente un radical de formule (II), (III), (IV), (V) ou (VI) suivantes :

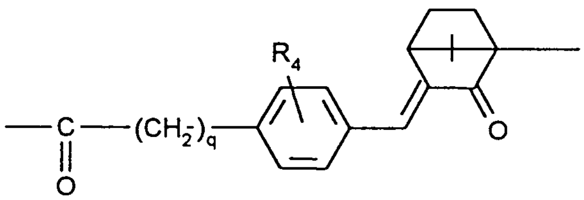

(II)

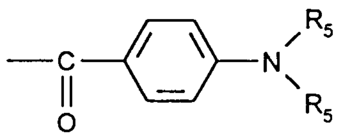

(III)

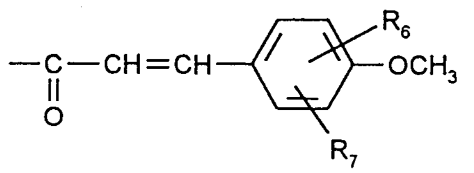

(IV)

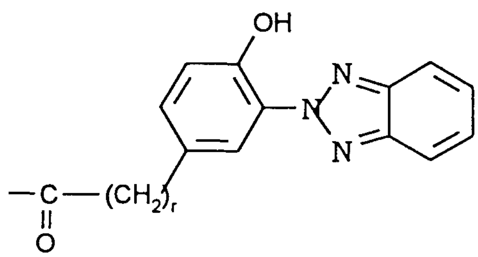

(V)

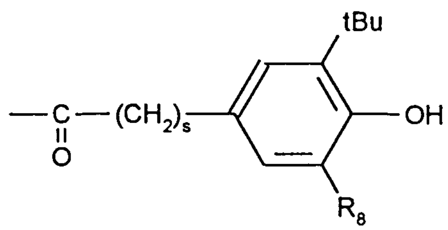

(VI)

ou leur mélange,
formules dans lesquelles $R_4$, $R_6$ et $R_7$ représentent un atome d'hydrogène ou un radical alkyle en $C_1$-$C_6$ ou un radical alkoxy en $C_1$-$C_{12}$, $R_5$ représente un radical alkyle en $C_1$-$C_{12}$, q et r représentent un nombre entier égal à 0 ou 1, $R_8$ représente H ou un radical alkyle en $C_1$-$C_8$ ou un radical alkoxy en $C_1$-$C_4$, s est un nombre entier allant de 0 à 4,

* $OR_2$ représente un groupement, lié au squelette de base de la cyclodextrine, dans lequel $R_2$ représente un radical alkyle, linéaire ou ramifié, en $C_1$-$C_4$ ou un radical hydroxyalkyle en $C_2$-$C_4$, l'hydrogène du groupement hydroxyle dudit radical hydroxyalkyle pouvant être substitué par un autre groupement hydroxyalkyle en $C_1$-$C_4$ ou par un radical $R_1$ ou leur mélange,

l, m et n sont des valeurs statistiques telles que m représente le nombre de groupements $OR_1$ liés directement au squelette de base de la cyclodextrine et est une valeur statistique, différente de 0,
n représente le nombre de groupements $OR_2$ liés directement au squelette de base de la cyclodextrine et est une valeur statistique, différente de 0,
l est une valeur statistique telle que (l + m + n ) est égal à 18, 21, 24 selon que CD est respectivement le squelette de base de l'α-, la β- ou la γ-cyclodextrine.

Les composés de formule (I) portant des radicaux de formule (II) à (V) ont une activité filtrante du rayonnement solaire et ceux portant un radical de formule (VI) ont une activité antioxydante. Les composés de formule (I) portant à la fois au moins un radical de formule (II) à (V) et au moins un radical de formule (VI) ont une activité filtrante et antioxydante.

De préférence, $R_2$ désigne un radical méthyle ou hydroxypropyle, l'hydrogène du groupement hydroxyle du radical hydroxypropyle pouvant être substitué par un autre groupement hydroxypropyle ou par un radical $R_1$.

Lorsque CD représente le squelette de base de l'α-cyclodextrine, n est une valeur allant de 3 à 5, et le nombre total p de groupements $R_1$ va de 2,5 à 14.

Lorsque CD représente le squelette de base de la β-cyclodextrine, n est une valeur allant de 3 à 8, et en particulier une valeur égale à 4,2 ou 6,3, et le nombre total p de groupements $R_1$ va de 2,5 à 16.

Lorsque CD représente le squelette de base de la γ-cyclodextrine, n est une valeur allant de 4 à 8, et le nombre total p de groupements $R_1$ va de 2,5 à 18.

La valeur de p peut être déterminée par l'indice de saponification ou par spectre RMN.

De préférence, CD est la β-cyclodextrine.

n est de préférence une valeur statistique allant de 4 à 7.

Les composés selon l'invention sont pratiquement insolubles dans l'eau, la solubilité diminuant en fonction du nombre de groupement $OR_2$. Ils sont légèrement solubles dans les huiles telles que l'adipate de diisopropyle, mais plus solubles dans les alcools tels que l'éthanol ou l'isopropanol. Plus le nombre de groupements $OR_2$ présents dans lesdits composés est élevé, plus la solubilité des composés dans les alcools diminue.

La présente invention a également pour objet le procédé de préparation des composés de formule (I). Ce procédé consiste à faire réagir dans un solvant (i) une cyclodextrine substituée de formule $CD(OR_2)_n(OH)_{l'}$, $OR_2$ représentant un groupement lié au squelette de base de la cyclodextrine, dans lequel $R_2$ désigne un radical alkyle, linéaire ou ramifié, en $C_1$-$C_4$ ou un radical hydroxyalkyle en $C_2$-$C_4$, l'hydrogène du groupement hydroxyle du radical hydroxyalkyle pouvant être substitué par un autre groupement hydroxyalkyle, n étant une valeur statistique ayant la même signification que ci-dessus, l' est une valeur statistique non nulle telle que l' + n est égal à 18, 21 ou 24 selon que CD représente le squelette de base de l'α-, la β- ou la γ-cyclodextrine, avec (ii) p moles d'un chlorure d'acide de formule Cl-$R_1$, $R_1$ désignant un radical de formule (II), (III), (IV), (V) ou (VI) tel que défini précédemment, p ayant la même signification que ci-dessus.

La réaction peut être effectuée dans un solvant solvant organique, notamment anhydre, tel que la pyridine ou le diméthyl formamide.

La réaction peut être effectuée à une température comprise entre 0 °C et 80 °C et de préférence entre 10 °C et 30 °C.

La réaction peut être effectuée en présence d'un catalyseur d'acylation. Celui-ci peut être notamment la para-N, N-diméthylamino pyridine.

La présente invention a également pour objet une composition comprenant au moins un composé de formule (I) tel que défini ci-dessus.

De préférence, la composition comprend un milieu cosmétiquement acceptable et constitue, en particulier, une composition destinée à protéger la peau et/ou les cheveux du rayonnement UV et/ou solaire.

Dans les compositions selon l'invention, les dérivés de formule (I) peuvent être présents à une concentration pouvant aller de 0,5 % à 10 % et de préférence de 0,5 % à 5 % en poids par rapport au poids total de la composition.

Ces compositions peuvent contenir les ingrédients habituellement utilisés en cosmétique. Ainsi, elles peuvent contenir au moins un constituant choisi parmi les corps gras, les épaississants, les esters d'acides gras, les esters d'acides gras et de glycérol, les silicones (volatiles ou non, fonctionnalisés ou non), les alcools gras, les tensioactifs, les parfums, les conservateurs, les filtres solaires, les protéines, les vitamines, les polymères, les huiles organiques ou inorganiques, les pigments, les charges, et tout autre additif classiquement utilisé dans le domaine cosmétique.

Les compositions de l'invention peuvent se présenter sous toute forme de solutions ou de dispersions des dérivés tels que définis ci-dessus, éventuellement vésiculaires.

Toutes ces compositions sont préparées selon les méthodes usuelles connues de l'homme de l'art.

L'invention a également pour objet l'utilisation des composés de formule (I) tels que définis ci-dessus dans, ou pour la fabrication de, une composition cosmétique. Elle a aussi pour objet l'utilisation des composés de formule (I) comme agent filtrant le rayonnement solaire et/ou comme agent antioxydant.

On va maintenant donner à titre d'illustration et sans aucun caractère limitatif plusieurs exemples de préparation de composés selon l'invention ainsi que des exemples de compositions cosmétiques les contenant.

Dans les exemples de préparation, E 1% correspond à la densité optique d'une solution de 1 g de composé dans 100 g de solvant.

**Exemple 1** : Préparation d'un composé de formule (I) dans laquelle $R_1$ est un radical de formule (II) pour laquelle q = 0, $R_4$ = H,
$R_2$ est un radical hydroxypropyle, n = 4,2, p = 3

A une solution de 1,72 g (1,25 mmole) d'hydroxypropyle β-cyclodextrine (n=4,2) vendu sous la dénomination RHODOCAP HP de la société RHÔNE-POULENC, dans 20 ml de pyridine anhydre, on ajoute à 20 °C par portion pendant

20 minutes, sous atmosphère d'azote, 1,74 g (5,75 mmole) de chlorure d'acide benzylidène camphre 4'-benzoïque. Après 40 heures d'agitation à température ambiante, le mélange réactionnel est évaporé sous pression réduite. Le résidu est ensuite repris dans de l'éther éthylique, filtré, lavé à l'eau et séché. Après purification sur colonne de silice (éluant dichlorométhane/ méthanol : 95/5), on obtient 2, 2 g d'une poudre jaune pâle ayant un point de ramolissement de 240 °C.

Infra-rouge : bande ester à 1715 cm-1
UV (éthanol absolu) ; $\lambda_{max}$ = 300 nm; E 1 % = 435

**Exemple 2** : Préparation d'un composé de formule (I) dans laquelle $R_1$ est un radical de formule (III) pour laquelle $R_5$ = méthyle,
$R_2$ est un radical hydroxypropyle, n = 6,3, p = 5,7

Le composé est préparé selon le même mode opératoire de l'exemple 1, en utilisant :

- 3 g (2 mmole) d'hydroxypropyle β-cyclodextrine (n = 6,3) vendu par la société ALDRICH
- 2,55 g (13,9 mmole) de chlorure de l'acide para N,N-diméthyl amino benzoïque

On laisse sous agitation pendant 24 heures. Après l'élimination du solvant, la gomme obtenue est reprise dans de l'éther diisopropylique, filtrée, lavée à l'eau et le solide obtenu est séché. Celui-ci est lavé à l'éthanol chaud. Après cristallisation, on obtient 1,6 g d'une poudre jaune pâle ayant un point de ramollissement de 210 °C.

Infra-rouge : bande ester à 1715 cm-1
UV (éthanol absolu) : $\lambda_{max}$ = 311 nm; E 1 % = 635

**Exemple 3** : Préparation d'un composé de formule (I) dans laquelle $R_1$ est un radical de formule (IV) pour laquelle $R_6$ = $R_7$ = H,
$R_2$ est un radical hydroxypropyle, n = 4,2, p = 4,5

Le composé est préparé selon le même mode opératoire de l'exemple 1, en utilisant :

- 1,25 g (0,9 mmole) d'hydroxypropyle β-cyclodextrine (n = 4,2)
- 0,825 g (4,2 mmole) de chlorure de l'acide para méthoxy cinnamique

Après purification du résidu sur colonne de silice (éluant dichlorométhane/ méthanol : 90/10), on obtient 1,1 g d'une poudre jaune pâle ayant un point de ramollissement de 67 °C.

Infra-rouge : bande ester à 1715 cm-1
UV (éthanol absolu) : $\lambda_{max}$ = 307 nm; E 1 % = 450

**Exemple 4** : Préparation d'un composé de formule (I) dans laquelle $R_1$ est un radical de formule (IV) pour laquelle $R_6$ = $R_7$ = H,
$R_2$ est un radical méthyle, n = 14,6, p = 2,9

Le composé est préparé selon le même mode opératoire de l'exemple 1, en utilisant :

- 2 g (15 mmole) de méthyl β-cyclodextrine (n = 14,6) vendu sous la dénomination PMCD par la société ORSAN
- 2,06 g (10,5 mmole) de chlorure de l'acide para méthoxy cinnamique

Après agitation pendant 100 heures, on ajoute au milieu réactionnel 20 ml d'eau et 20 ml de dichlorométhane. On récupère la phase organique qui est évaporée. La gomme obtenue est reprise dans de l'éther isopropylique, filtrée, lavée à l'eau. Après purification du résidu sur colonne de silice (éluant dichlorométhane/ méthanol : 98/2), on obtient 1,5 g d'une poudre jaune pâle ayant un point de fusion de 185-187 °C.

Infra-rouge : bande ester à 1715 $cm^{-1}$
UV (éthanol absolu) : $\lambda_{max}$ = 309 nm; E 1 % = 350

**Exemple 5** : Préparation d'un composé de formule (I) dans laquelle $R_1$ est un radical de formule (VI) pour laquelle $R_8$ = tBu, s = 0
$R_2$ est un radical hydroxypropyle, n = 4,2, p = 3,8

Le composé est préparé selon le même mode opératoire de l'exemple 1, en utilisant :

- 27,6 g (20 mmole) d'hydroxypropyl β-cyclodextrine (n = 4,2)
- 24,8 g (92,4 mmole) de chlorure de l'acide 3,5-ditert butyl 4-hydroxybenzoïque

Après purification du résidu sur colonne de silice (éluant dichlorométhane/ méthanol : 98/2 puis gradient jusqu'à 85/15), on obtient 27,2 g d'une poudre blanc cassé ayant un point de ramollissement de 255 °C.
Infra-rouge : bande ester à 1710 $cm^{-1}$

**Exemple 6** : Préparation d'un composé de formule (I) dans laquelle $R_1$ est un radical de formule (VI) pour laquelle $R_8$ = tBu, s = 0
$R_2$ est un radical hydroxypropyle, n = 4,2, p = 15

Le composé est préparé selon le même mode opératoire de l'exemple 1, en utilisant :

- 2,78 g (2 mmole) d'hydroxypropyl β-cyclodextrine (n = 4,2)
- 12,5 g (46,5 mmole) de chlorure de l'acide 3,5-ditert butyl 4-hydroxybenzoïque

Après agitation pendant 40 heures, on verse le milieu réactionnel dans 150 ml d'eau glacée, on filtre et lave à l'eau le solide obtenu. Après purification du résidu sur colonne de silice (éluant dichlorométhane/ méthanol : 98/2 puis gradient jusqu'à 85/15), on obtient 4,3 g d'une poudre blanc cassé ayant un point de ramollissement de 215 °C.
Infra-rouge : bande ester à 1710 $cm^{-1}$

**Exemple 7** : Préparation d'un composé de formule (I) dans laquelle $R_1$ est un radical de formule (VI) pour laquelle $R_8$ = tBu, s = 2
$R_2$ est un radical hydroxypropyle, n = 4,2, p = 3

Le composé est préparé selon le même mode opératoire de l'exemple 1, en utilisant :

- 27,6 g (0,02 mole) d'hydroxypropyl β-cyclodextrine (n = 4,2)
- 27,4 g (0,092 mole) de chlorure de l'acide 3,5-ditert butyl 4-hydroxyphényl propionique.

Après purification du résidu sur colonne de silice (éluant dichlorométhane/ méthanol : 98/2 puis gradient jusqu'à 85/15), on obtient 25,6 g d'une poudre blanc cassé ayant un point de ramollissement de 170 °C.

Infra-rouge : bande ester à 1710 $cm^{-1}$

**Exemple 8** : Préparation d'un composé de formule (I) dans laquelle $R_1$ est un radical de formule (VI) pour laquelle $R_8$ = tBu, s = 0
$R_2$ est un radical méthyle, n = 14,6, p = 3,6

Le composé est préparé selon le même mode opératoire de l'exemple 1, en utilisant :

- 0,71 g (0,525 mmole) de méthyl β-cyclodextrine (n = 14,6)
- 1 g (3,72 mmole) de chlorure de l'acide 3,5-ditert butyl 4-hydroxybenzoïque

Après élimination du solvant, le résidu est lavé à l'eau, extrait au dichlorométhane. Après évaporation, le résidu est purifié sur colonne de silice (éluant dichlorométhane), on obtient 0,8 g d'une poudre blanc cassé ayant un point de ramollissement de 190 °C.
Infra-rouge : bande ester à 1710 $cm^{-1}$

**<u>Exemple 9</u>** : Préparation d'un composé de formule (I) dans laquelle $R_1$ est un mélange de radical de formule (VI) pour laquelle $R_8$ = tBu, s = 2 et de radical de formule (IV) dans laquelle $R_6$ = $R_7$ = H
$R_2$ est un radical hydroxypropyle, n = 4,2, p = 6,4

p antioxydant = 2,6
p filtre = 3,8

A une solution de 2,16 g (1 mmole) de composé de l'exemple 7 dans 15 ml de pyridine anhydre, on ajoute à 20 °C en 5 minutes 0,9 g (4,6 mmole) de chlorure de l'acide p-méthoxy cinnamique. Après agitation pendant 40 heures, sous atmosphère d'azote, on élimine le solvant sous pression réduite. Le résidu est repris dans de l'éther isopropylique puis dans de l'eau et est filtré. Après purification du résidu sur colonne de silice (éluant dichlorométhane/méthanol : 98/2 puis gradient jusqu'à 85/15), on obtient 1,7 g d'une poudre blanc cassé ayant un point de ramollissement de 160 °C.

Infra-rouge : bande ester à 1710 $cm^{-1}$
UV (éthanol absolu) : $\lambda_{max}$ = 310 nm; E 1 % = 340

**<u>Exemple 10</u> :**

On a préparé une composition antisolaire de formulation suivante :

| | |
|---|---|
| - Mélange d'alcool cétylstéarylique et d'alccol cétylstéarylique oxyéthyléné à 33 mole d'oxyde d'éthylène (80/20) vendu sous la dénomination "DEHSCONET 390" par la société TENSIA | 7 g |
| - Stéarate de glycéryle | 2 g |
| - Alcool cétylique | 1,5 g |
| - Diméthicone vendue sous la dénomination "SILBIONE huile 70047 V 300" par la société RHONE-POULENC | 1,5 g |
| - Huile de vaseline | 15 g |
| - Propylène glycol | 20 g |
| - Composé de l'exemple 3 | 5 g |
| - p-hydroxybenzoate de butyle | 0,2 g |
| - imidazolidinyl urée | 0,2 g |
| - Eau déminéralisée qsp | 100 g |

**Revendications**

**1.** Dérivés de cyclodextrines caractérisés par le fait qu'ils répondent à la formule générale (I) suivante :

$$CD\ (OH)_l\ (OR_1)_m(OR_2)_n \qquad (I)$$

dans laquelle

* CD représente le squelette de base de l'α-, β- ou γ-cyclodextrine sans les groupements hydroxyle,
* $OR_1$ représente un groupement, lié au squelette de base de la cyclodextrine, dans lequel $R_1$ représente un radical de formule (II), (III), (IV), (V) ou (VI) suivantes :

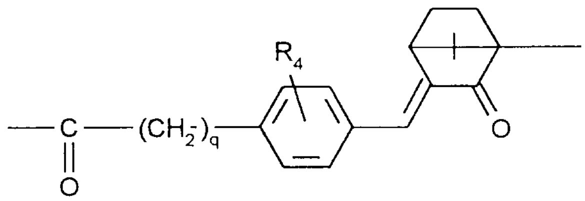

(II)

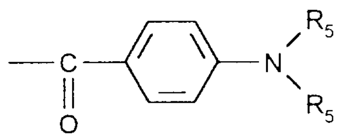

(III)

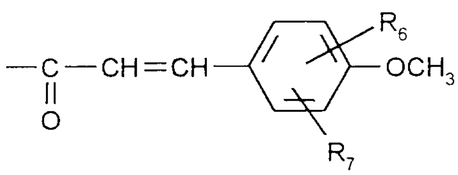

(IV)

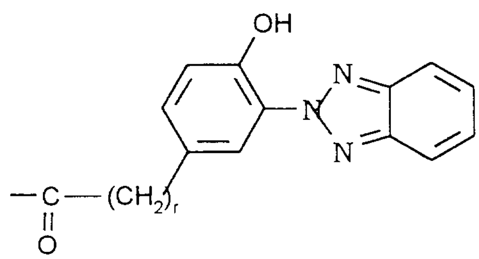

(V)

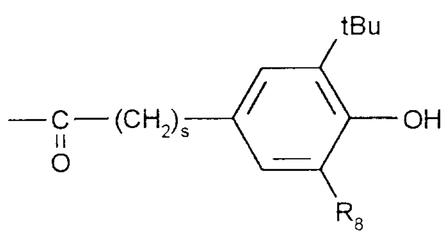

(VI)

ou leur mélange,
dans lesquelles $R_4$, $R_6$ et $R_7$ représentent un atome d'hydrogène ou un radical alkyle en $C_1$-$C_6$ ou un radical alkoxy en $C_1$-$C_{12}$, $R_5$ représente un radical alkyle en $C_1$-$C_{12}$ q et r représentent un nombre entier égal à 0 ou 1, $R_8$ représente H ou un radical alkyle en $C_1$-$C_8$ ou un radical alkoxy en $C_1$-$C_4$, s est un nombre entier allant de 0 à 4,

* $OR_2$ représente un groupement, lié au squelette de base de la cyclodextrine, dans lequel $R_2$ représente un radical alkyle, linéaire ou ramifié, en $C_1$-$C_4$ ou un radical hydroxyalkyle en $C_2$-$C_4$ l'hydrogène du groupement hydroxyle dudit radical hydroxyalkyle pouvant être substitué par un autre groupement hydroxyalkyle ou par un radical $R_1$ ou leur mélange,

l, m et n sont des valeurs statistiques telles que m représente le nombre de groupements $OR_1$ liés directement au squelette de base de la cyclodextrine et est une valeur statistique, différente de 0,

n représente le nombre de groupements $OR_2$ liés directement au squelette de base de la cyclodextrine et est une valeur statistique, différente de 0 ;

l est une valeur statistique telle que ( l + m + n ) est égal à 18, 21, 24 selon que CD est respectivement le

squelette de base de l'α-, la β- ou la γ-cyclodextrine ;
sous réserve que :

(i) lorsque CD représente le squelette de base de l'α-cyclodextrine, n va de 3 à 5 et p désignant nombre total de groupements $R_1$ varie de 2,5 à 14 ;
(ii) lorsque CD représente le squelette de base de la β-cyclodextrine, n va de 3 à 8 et p désignant nombre total de groupements $R_1$ varie de 2,5 à 16 ;
(iii) lorsque CD représente le squelette de base de la γ-cyclodextrine, n va de 4 à 8 et p désignant nombre total de groupements $R_1$ varie de 2,5 à 18.

**2.** Dérivés de cyclodextrine selon la revendication 1, caractérisés par le fait que $R_2$ désigne un radical méthyle ou hydroxypropyle, l'hydrogène du goupement hydroxyle du radical hydroxypropyle pouvant être substitue par un autre groupement hydroxypropyle ou par un radical $R_1$.

**3.** Dérivés de cyclodextrine selon l'une des revendications 1 et 2, caractérisés par le fait que CD est la β-cyclodextrine.

**4.** Procédé de préparation des dérivés de cyclodextrine de formule (I), caractérisé par le fait que l'on fait réagir en solution dans un solvant organique (i) une cyclodextrine substituée de formule $CD(OR_2)_n(OH)_{l'}$, avec
(ii) p moles d'un chlorure d'acide de formule $Cl\text{-}R_1$ ,
étant entendu que :

n et p sont des valeurs statistiques non nulles,
n va de 3 à 5, et p va de 2,5 à 14 lorsque CD représente le squelette de base de l'α-cyclodextrine,
n va de 3 à 8, et p va de 2,5 à 16 lorsque CD représente le squelette de base de la β-cyclodextrine,
n va de 4 à 8, et p va de 2,5 à 18 lorsque CD représente le squelette de base de la γ-cyclodextrine
l' est une valeur statistique non nulle telle que l' + n est égal à 18, 21 ou 24 selon que CD représente le squelette de base de l'α-, la β- ou la γ-cyclodextrine,
$OR_2$ et $R_1$ ont la signification donnée dans les revendications précédentes.

**5.** Composition caractérisée par le fait qu'elle comprend au moins un composé de formule (I) tel que défini à l'une quelconque des revendications 1 à 4.

**6.** Composition selon la revendication 7, caractérisée par le fait qu'elle comprend un milieu cosmétiquement acceptable.

**7.** Composition selon la revendication 8 ou 9, caractérisée par le fait que les composés de formule (I) sont présents à une concentration allant de 0,5 % à 10 % en poids par rapport au poids total de la composition.

**8.** Composition selon l'une quelconques des revendications 7 à 10, caractérisée par le fait qu'il s'agit d'une composition destinée à protéger la peau et/ou les cheveux du rayonnement UV.

**9.** Utilisation d'un composé de formule (I) tel que défini à l'une quelconque des revendications 1 à 3, dans, ou pour la préparation de, une composition cosmétique.

**10.** Utilisation selon la revendication 12, caractérisée par le fait que ladite composition cosmétique est destinée à protéger la peau et/ou les cheveux du rayonnement UV et/ou solaire.

**11.** Utilisation d'un composé de formule (I) tel que défini à l'une quelconque des revendications 1 à 3 comme agent filtrant et/ou comme agent antioxydant.

## Claims

**1.** Cyclodextrin derivatives, characterized in that they correspond to the general formula (I) below:

$$CD(OH)_l(OR_1)_m(OR_2)_n \qquad (I)$$

in which:

- * CD represents the basic skeleton of α-, β- or γ-cyclodextrin without the hydroxyl groups,
- * $OR_1$ represents a group, attached to the basic skeleton of the cyclodextrin, in which $R_1$ represents a radical of formula (II), (III), (IV), (V) or (VI) below:

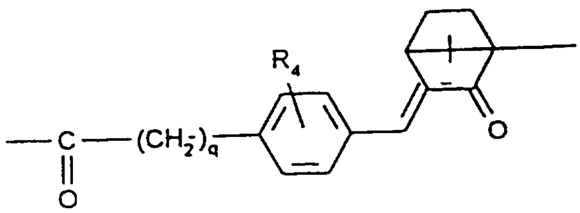

(II)

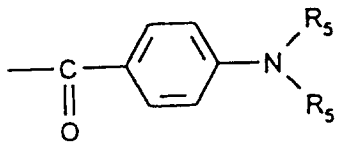

(III)

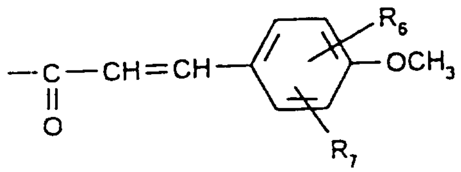

(IV)

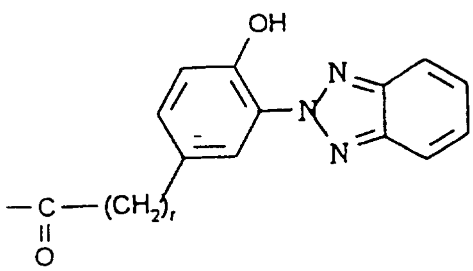

(V)

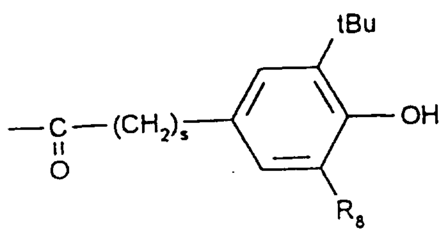

(VI)

or a mixture thereof,
in which formulae $R_4$, $R_6$ and $R_7$ represent a hydrogen atom or a $C_1$-$C_6$alkyl radical or a $C_1$-$C_{12}$alkoxy radical, $R_5$ represents a $C_1$-$C_{12}$alkyl radical, q and r represent an integer equal to 0 or 1,
$R_8$ represents H or a $C_1$-$C_8$alkyl radical or a $C_1$-$C_4$alkoxy radical, s is an integer ranging from 0 to 4,

- * $OR_2$ represents a group, attached to the basic skeleton of the cyclodextrin, in which $R_2$ represents a linear or branched $C_1$-$C_4$alkyl radical or a $C_2$-$C_4$hydroxyalkyl radical, it being possible for the hydrogen of the hydroxyl group of the said hydroxyalkyl radical to be substituted with another hydroxyalkyl group or with a radical $R_1$ or a mixture thereof,

l, m and n are statistical values such that m represents the number of groups $OR_1$ attached directly to the basic skeleton of the cyclodextrin and is a statistical value other than 0,
n represents the number of groups $OR_2$ attached directly to the basic skeleton of the cyclodextrin and is a statistical value other than 0,
l is a statistical value such that (l+m+n) is equal to 18, 21, 24 depending on whether CD is the basic skeleton of α-, β- or γ-cyclodextrin respectively,
with the proviso that:

(i) when CD represents the basic skeleton of α-cyclodextrin, n ranges from 3 to 5 and p, denoting the

total number of groups $R_1$, ranges from 2.5 to 14;

(ii) when CD represents the basic skeleton of β-cyclodextrin, n ranges from 3 to 8 and p, denoting the total number of groups $R_1$, ranges from 2.5 to 16;

(iii) when CD represents the basic skeleton of γ-cyclodextrin, n ranges from 4 to 8 and p, denoting the total number of groups $R_1$, ranges from 2.5 to 18.

2. Cyclodextrin derivatives according to Claim 1, characterized in that $R_2$ denotes a methyl or hydroxypropyl radical, it being possible for the hydrogen of the hydroxyl group of the hydroxypropyl radical to be substituted with another hydroxypropyl group or with a radical $R_1$.

3. Cyclodextrin derivatives according to either of Claims 1 and 2, characterized in that CD is β-cyclodextrin.

4. Process for the preparation of the cyclodextrin derivatives of formula (I), characterized in that (i) a substituted cyclodextrin of formula $CD(OR_2)_n(OH)_{l'}$ is reacted, in solution in an organic solvent, with (ii) p moles of an acid chloride of formula $Cl\text{-}R_1$,
it being understood that:

   n and p are non-zero statistical values,
   n ranges from 3 to 5 and p ranges from 2.5 to 14 when CD represents the basic skeleton of α-cyclodextrin,
   n ranges from 3 to 8 and p ranges from 2.5 to 16 when CD represents the basic skeleton of β-cyclodextrin,
   n ranges from 4 to 8 and p ranges from 2.5 to 18 when CD represents the basic skeleton of γ-cyclodextrin,
   l' is a non-zero statistical value such that l'+n is equal to 18, 21 or 24 depending on whether CD represents the basic skeleton of α-, β- or γ-cyclodextrin,
   $OR_2$ and $R_1$ have the meaning given in the preceding claims.

5. Composition characterized in that it comprises at least one compound of formula (I) as defined in any one of Claims 1 to 4.

6. Composition according to Claim 5, characterized in that it comprises a cosmetically acceptable medium.

7. Composition according to Claim 5 or 6, characterized in that the compounds of formula (I) are present at a concentration ranging from 0.5% to 10% by weight relative to the total weight of the composition.

8. Composition according to any one of Claims 5 to 7, characterized in that it is a composition intended to protect the skin and/or the hair against UV radiation.

9. Use of a compound of formula (I) as defined in any one of Claims 1 to 3, in, or for the preparation of, a cosmetic composition.

10. Use according to Claim 9, characterized in that the said cosmetic composition is intended to protect the skin and/or the hair against UV and/or solar radiation.

11. Use of a compound of formula (I) as defined in any one of Claims 1 to 3, as a screening agent and/or as an antioxidant.

**Patentansprüche**

1. Cyclodextrinderivate, dadurch gekennzeichnet, daß sie der folgenden allgemeinen Formel (I) entsprechen:

$$CD(OH)_l(OR_1)_m(OR_2)_n \qquad (I),$$

worin bedeuten:

* CD das Grundgerüst von α-Cyclodextrin, β-Cyclodextrin oder γ-Cyclodextrin ohne die Hydroxygruppen,

* $OR_1$ eine an das Grundgerüst des Cyclodextrin gebundene Gruppe, worin $R_1$ eine Gruppe der folgenden Formeln (II), (III), (IV), (V) oder (VI):

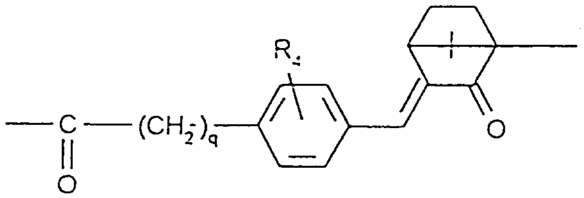

(II)

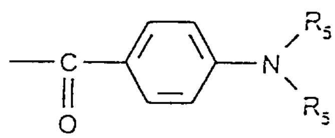

(III)

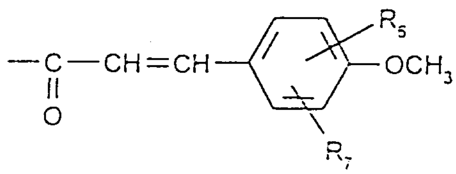

(IV)

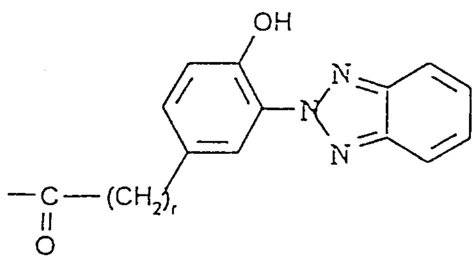

(V)

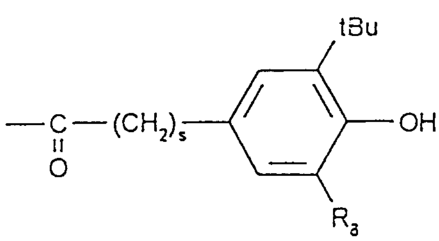

(VI)

oder ein Gemisch dieser Gruppen bedeutet,
wobei in den Formeln bedeuten:

- $R_4$, $R_6$ und $R_7$ Wasserstoff, $C_{1-6}$-Alkyl oder $C_{1-12}$-Alkoxy,
- $R_5$ $C_{1-12}$-Alkyl,
- q und r Null oder 1,
- $R_8$ Wasserstoff, $C_{1-8}$-Alkyl oder $C_{1-4}$-Alkoxy und
- s Null oder eine ganze Zahl von 1 bis 4,

* $OR_2$ eine an das Grundgerüst des Cyclodextrins gebundene Gruppe, worin $R_2$ eine geradkettige oder verzweigte $C_{1-4}$-Alkylgruppe oder eine $C_{2-4}$-Hydroxyalkylgruppe bedeutet, wobei der Wasserstoff der Hydroxygruppe dieser Hydroxyalkylgruppe durch eine weitere Hydroxyalkylgruppe, eine Gruppe $R_1$ oder ein Gemisch dieser Gruppen substituiert sein kann, und

* l, m und n statistische Werte, von denen:

- m die Anzahl von Gruppen $OR_1$, die direkt an das Grundgerüst des Cyclodextrins gebunden sind, bedeutet und ein statistischer Wert ist, der von Null verschieden ist,
- n die Anzahl von Gruppen $OR_2$, die direkt an das Grundgerüst des Cyclodextrins gebunden sind, darstellt und ein statistischer Wert ist, der von Null verschieden ist, und
- l einen solchen statistischen Wert, daß (l + m + n ) gleich 18, 21 oder 24 ist, je nachdem, ob CD jeweils das Grundgerüst des α-Cyclodextrins, des β-Cyclodextrins bzw. des γ-Cyclodextrins bedeutet, mit der Maßgabe, daß:

(i) n einen Wert von 3 bis 5 aufweist und p, das die Gesamtzahl von Gruppen $R_1$ bezeichnet, im Bereich von 2,5 bis 14 liegt, wenn CD das Grundgerüst des α-Cyclodextrins bedeutet,
(ii) n einen Wert von 3 bis 8 aufweist und p, das die Gesamtzahl von Gruppen $R_1$ bezeichnet, im Bereich von 2,5 bis 16 liegt, wenn CD das Grundgerüst des β-Cyclodextrins bedeutet, und
(iii)n einen Wert von 4 bis 8 aufweist und p, das die Gesamtzahl von Gruppen $R_1$ bezeichnet, im Bereich von 2,5 bis 18 liegt, wenn CD das Grundgerüst des γ-Cyclodextrins bedeutet.

**2.** Cyclodextrinderivate nach Anspruch 1, dadurch gekennzeichnet, daß $R_2$ eine Methylgruppe oder eine Hydroxypropylgruppe bedeutet, wobei der Wasserstoff der Hydroxygruppe der Hydroxypropylgruppe durch eine weitere Hydroxypropylgruppe oder eine Gruppe $R_1$ substituiert sein kann.

**3.** Cyclodextrinderivate nach Anspruche 1 oder 2, dadurch gekennzeichnet, daß CD β-Cyclodextrin ist.

**4.** Verfahren zur Herstellung der Cyclodextrinderivate der Formel (I), dadurch gekennzeichnet, daß in Lösung in einem organischen Lösungsmittel (i) ein substituiertes Cyclodextrin der Formel $CD(OR_2)_n(OH)_{l'}$ mit (ii) p mol eines Säurechlorids der Formel $Cl-R_1$ umgesetzt wird,
wobei bedeuten:

- n und p statistische Werte, die nicht gleich Null sind,
- n einen Wert im Bereich von 3 bis 5 und p einen Wert im Bereich von 2,5 bis 14, wenn CD das Grundgerüst des α-Cyclodextrins bedeutet,
- n einen Wert im Bereich von 3 bis 8 und p einen Wert im Bereich von 2,5 bis 16, wenn CD das Grundgerüst des β-Cyclodextrins bedeutet,
- n einen Wert im Bereich von 4 bis 8 und p einen Wert im Bereich von 2,5 bis 18, wenn CD das Grundgerüst des γ-Cyclodextrins bedeutet,
- l' einen statistischen Wert, der nicht gleich Null und so ist, daß l' + n gleich 18, 21 oder 24 ist, je nachdem, ob CD das Grundgerüst des α-Cyclodextrins, des β-Cyciodextrins oder des γ-Cyclodextrins bedeutet, und
- $OR_2$ und $R_1$ dasselbe wie in den vorangehenden Ansprüchen angegeben.

**5.** Zusammensetzung, dadurch gekennzeichnet, daß sie mindestens eine in einem der Ansprüche 1 bis 4 definierte Verbindung der Formel (I) enthält.

**6.** Zusammensetzung nach Anspruch 5, dadurch gekennzeichnet, daß sie ein kosmetisch akzeptables Medium enthält.

**7.** Zusammensetzung nach Anspruch 6, dadurch gekennzeichnet, daß die Verbindungen der Formel (I) in einer Konzentration von 0,5 bis 10 Gew.-%, bezogen auf das Gesamtgewicht der Zusammensetzung, vorliegen.

**8.** Zusammensetzung nach einem der Ansprüche 5 bis 7, dadurch gekennzeichnet, daß es sich um eine Zusammensetzung handelt, die zum Schutz der Haut und/oder der Haare gegen UV-Strahlung bestimmt ist.

**9.** Verwendung einer in einem der Ansprüche 1 bis 3 definierten Verbindung der Formel (I) in einer kosmetischen Zusammensetzung oder zur Herstellung einer kosmetischen Zusammensetzung.

**10.** Verwendung nach Anspruch 9, dadurch gekennzeichnet, daß die kosmetische Zusammensetzung zum Schutz der Haut und/oder der Haare gegen UV-Strahlung und/oder Sonnenstrahlung bestimmt ist.

11. Verwendung einer in einem der Ansprüche 1 bis 3 definierten Verbindung der Formel (I) als Filter und/oder als Antioxidans.